(19) Europäisches Patentamt
European Patent Office
Office européen des brevets

(11) Veröffentlichungsnummer : **0 406 742 B1**

(12) **EUROPÄISCHE PATENTSCHRIFT**

(45) Veröffentlichungstag der Patentschrift : **21.04.93 Patentblatt 93/16**

(51) Int. Cl.[5] : **C07C 69/003,** C07C 67/00, C07C 31/137, C07C 29/00

(21) Anmeldenummer : **90112541.9**

(22) Anmeldetag : **30.06.90**

(54) **Verfahren zur Herstellung von cis-Dihydronopol.**

(30) Priorität : **07.07.89 DE 3922386**

(43) Veröffentlichungstag der Anmeldung : **09.01.91 Patentblatt 91/02**

(45) Bekanntmachung des Hinweises auf die Patenterteilung : **21.04.93 Patentblatt 93/16**

(84) Benannte Vertragsstaaten : **AT BE CH DE DK ES FR GB GR IT LI LU NL SE**

(56) Entgegenhaltungen :
**FR-A- 2 097 031**
**FR-A- 2 424 244**

(73) Patentinhaber : **Kali-Chemie Pharma GmbH**
**Hans-Böckler-Allee 20 Postfach 220**
**W-3000 Hannover 1 (DE)**

(72) Erfinder : **Heitmann, Walter**
**3711 Robinson Walk Ct.**
**Marietta, GA 30068 (US)**
Erfinder : **Mätzel, Uwe**
**Habichtshorst 9**
**W-3167 Burgdorf (DE)**

(74) Vertreter : **Lauer, Dieter, Dr.**
**c/o Kali-Chemie Aktiengesellschaft Postfach 220**
**W-3000 Hannover 1 (DE)**

EP 0 406 742 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein Verfahren zur Herstellung von cis-Dihydronopol (= 2-((1S,2S,5S)-6,6-Dimethylbicyclo[3.1.1]hept-2-yl)-äthanol) und dessen, niederen Carbonsäure- oder Benzoesäureestern. Dihydronopol ist ein 2-(6,6-Dimethylbicyclo[3.1.1]hept-2-yl)-äthanol der Formel A

(s. Formel A)

Die Formel A enthält in den Positionen 1, 2 und 5 des Ring-Gerüstes chirale Zentren, welche jeweils R- oder S-konfiguriert sein können, so daß die Substanzen in mehreren stereoisomeren Formen auftreten können.

Das Dihydronopol leitet sich von dem natürlichen Terpen (-)-β-Pinen (= (1S,5S)-6,6-Dimethyl-2-methylenbicyclo-[3.1.1]heptan) der Formel B

(s. Formel B)

ab, in welchem die chiralen Zentren in 1- und 5-Position S-konfiguriert sind. Dementsprechend besitzen auch im Dihydronopol die Zentren in 1- und 5-Position S-Konfiguration, während das Zentrum in S-Position 5-oder R-Konfiguration besitzen kann. So ist der Substituent in 2-Stellung im cis-Dihydronopol cis-ständig zur Dimethylmethylenbrücke und im trans-Dihydronopol dazu trans-ständig.

Dihydronopol ist aus dem französischen Patent Nr. 2097031 bekannt als Zwischenprodukt zur Herstellung von pharmakologisch aktiven Substanzen. Beispielsweise stellt das Dihydronopol ein Zwischenprodukt dar für die Herstellung des als Spasmolytikum im Handel befindlichen Pinaverium-bromids (Handelsname Dicetel[R] = N-(2-Brom-4,5-dimethoxybenzyl)-N-{2-[2-((1S,5S)-6,6-dimethylbicyclo[3.1.1]hept-2-yl)- äthoxy]-äthyl)-morpholiniumbromid). Zur Herstellung des Pinaveriumbromids wird das Dihydronopol zunächst nach der in dem französischen Patent Nr. 2097031 angegebenen Methode mit Morpholinoäthylchlorid umgesetzt und das erhaltene Reaktionsprodukt weiter nach der in dem französischen Patent Nr. 2097032 bzw. dem deutschen Patent Nr. 2137988 beschriebenen Methode mit 2-Brom-4,5-dimethoxybenzylbromid umgesetzt.

Gemäß dem französischen Patent Nr. 2079031 wird das Dihydronopol aus dem aus (-)-β-Pinen erhältlichen und z.B. aus J. Am. Chem. Soc. 68 (1946), Seite 638, bekannten Terpenalkohol (-)-Nopol (= 2-((1R,5S)-6,6-Dimethylbicyclo[3.1.1]hept-2-en-2-yl)-äthanol) der Formel III

(s. Formel III)

durch Hydrierung der Doppelbindung erhalten. Diese Hydrierung wird bei Temperaturen von 80-100 °C in Gegenwart eines Platinoxidkatalysator (= Adamskatalysator) durchgeführt oder auch in Gegenwart von Raney-Nickel oder Urushi-bara-Nickel. Gewünschtenfalls kann ein Lösungsmittel, beispielsweise Alkohol verwendet werden.

Bei der Hydrierung des (-)-Nopols zu Dihydronopol nach der in dem französischen Patent beschriebenen Methode entsteht ein Stereoisomerengemisch, welches neben einem Hauptanteil an cis-Verbindung auch einen deutlichen Anteil trans-Verbindung enthält. Beim weiteren Umsetzen des Dihydronopols zu pharmakologisch aktiven Verbindungen bleibt die Konfiguration am Ringgerüst erhalten, so daß auch die pharmakologisch aktiven Endprodukte Stereoisomerengemische darstellen. Sofern stereoisomerenreine Verbindungen gewünscht werden, müssen diese aus den Gemischen durch an sich bekannte aufwendige und verlustreiche Trennverfahren zunächst angereichert und schließlich isoliert werden.

In der Arzneimittelherstellung wird generell die Verwendung von möglichst reinen und auch sterisch einheitlichen Wirkstoffen angestrebt.

Der vorliegenden Erfindung liegt deshalb die Aufgabe zugrunde, ein verbessertes Verfahren zur diastereoselektiven Herstellung von cis-Dihydronopol und dessen Estern zu entwickeln.

Es wurde nun ein Verfahren gefunden, mit welchem cis-Dihydronopol in guten Ausbeuten und mit hoher Diastereoselektivität erhalten werden kann.

Gegenstand der Erfindung ist daher ein Verfahren zur Herstellung von 2-((1S,2S,5S)-6,6-Dimethylbicyclo-[3.1.1]hept-2-yl)-äthanolderivaten der allgemeinen Formel I

(s. Formel I)

worin der Substituent in 2-Stellung cis-ständig zur Dimethylmethylenbrücke ist und R Wasserstoff oder eine Acylgruppe $COR^1$, worin $R^1$ niederes Alkyl oder gegebenenfalls durch niederes Alkyl, niederes Alkoxy oder Hydroxy substituiertes Phenyl bedeutet, darstellt, dadurch gekennzeichnet, daß man Ester der allgemeinen Formel II

(s. Formel II)

worin $R^1$ obige Bedeutung besitzt, mit Wasserstoff in Gegenwart eines festen Platin- oder Platinoxidkatalysators oder Ruthenium/Kohle-Katalysators hydriert zu Verbindungen der allgemeinen Formel Ia

(s. Formel Ia)

worin $R^1$ obige Bedeutung besitzt, und aus den Verbindungen der Formel Ia gewünschtenfalls die $COR^1$-Gruppe abspaltet.

Sofern der Substituent R der Verbindungen der Formel I niedere Alkylgruppen enthält, können diese geradkettig oder verzweigt sein und vorzugsweise 1 bis 4, insbesondere 1 oder 2 Kohlenstoffatome enthalten. Für das erfindungsgemäße Verfahren eignet sich insbesondere der Einsatz von niederen Carbonsäureestern der Formel II beispielsweise ein Acetat oder Propionat, vorzugsweise das Acetat.

Das erfindungsgemäße Verfahren stellt eine heterogene Hydrierung in Gegenwart eines festen Platin- oder Platinoxidkatalysators oder Ruthenium/Kohle-Katalysators dar. Als Katalysator kann zum Beispiel handelsübliches Platinoxid verwendet werden, welches einen Platingehalt von 79-85 % besitzt und beispielsweise unter der Bezeichnung "Adamskatalysator" bekannt ist. Als Platinkatalysatoren können Trägerkatalysatoren, welche Platin auf einem organischen oder anorganischen Trägermaterial enthalten, oder Platinschwarz eingesetzt werden. Als Trägermaterialien für die Trägerkatalysatoren kommen an sich zur Herstellung von Katalysatorträgern bekannte Materialien in Betracht. So eignen sich beispielsweise Aktivkohle oder auch keramische Materialien beispielsweise Aluminiumoxide, Siliziumoxide und Alumosilikate. Der Platingehalt solcher Trägerkatalysatoren kann zwischen 1 und 10, insbesondere 4 bis 6 Gew.-%, bezogen auf das Gesamtgewicht des Katalysators, betragen. Als günstig erweisen sich zum Beispiel Platin/Kohle-Katalysatoren mit einem Platingehalt von 3 bis 7 Gew.-%. Geeignet sind auch Trägerkatalysatoren, welche Ruthenium auf einem Kohleträger enthalten. Als günstig erweisen sich z.B. Ruthenium/Kohle-Katalysatoren mit einem Rutheniumgehalt von 1-10, vorzugsweise 3-7, Gew.-%. Die zu verwendende Katalysatormenge kann je nach Art des eingesetzten Katalysators und nach angewendetem Wasserstoffdruck und der Reaktionszeit variieren. Beim Arbeiten mit einem Wasserstoffdruck von ca. 80 bar können im allgemeinen befriedigende Ausbeuten innerhalb von 3-4 Stunden bei Verwendung von Platinoxid erhalten werden mit Katalysatormengen von 1-15 g Platinoxid/Mol zu hydrierender Ausgangs verbindung der Formel II und bei Verwendung von Platin/Kohle erhalten werden mit 0,1 - 1 g Platinmetall/Mol Ausgangsverbindung entsprechenden Katalysatormengen. Bei Verwendung von Ruthenium/Kohle-Katalysatoren können z.B. beim Arbeiten mit 95 bar Wasserstoffdruck im allgemeinen befriedigende Ausbeuten innerhalb von 4-5 Stunden mit 0,2 - 1 g Ruthenium/Mol Ausgangsverbindung entsprechenden Katalysatormengen erzielt werden.

Die Hydrierung kann ohne Zusatz eines weiteren Lösungsmittels oder in Gegenwart eines organischen Lösungsmittels erfolgen. Als organische Lösungmittel eignen sich aliphatische, cycloaliphatische oder aromatische Kohlenwasserstoffe wie n-Hexan, Cyclohexan oder Toluol, offenkettige oder cyclische Äther wie Tetrahydrofuran, niedere Alkylester von niederen Carbonsäuren wie beispielsweise Essigsäureäthylester, niedere Ketone wie Aceton oder niedere geradkettige oder verzweigte Alkanole, beispielsweise niedere Alkohole mit 1 bis 4, insbesondere 1 bis 3 Kohlenstoffatomen, insbesondere Äthanol oder Isopropanol.

Die Hydrierung kann bei einem Wasserstoffdruck im Bereich von 1 bis 100 bar durchgeführt werden. Zweckmäßigerweise wird unter erhöhtem Druck beispielsweise bei einem Wasserstoffdruck zwischen 3 und 95, vorzugsweise 60 und 95 bar gearbeitet. Bei Verwendung von Platin- oder Platinoxidkatalysatoren kann im allgemeinen mit niedrigerem Wasserstoffdruck als bei der Verwendung von Rutheniumkatalysatoren gearbeitet werden.

Die Hydrierung kann bei Raumtemperatur oder leicht erhöhten Temperaturen, beispielsweise bei Temperaturen zwischen 10 und 60 °C, vorzugsweise 15 und 50, insbesondere 18 und 25 °C, durchgeführt werden. Die Reaktionszeit variiert je nach angewendetem Wasserstoffdruck, Temperatur, Katalysatortyp und Katalysatormenge. Sie kann beispielsweise zwischen 1 und 10 Stunden liegen. Im allgemeinen wird ein weitgehend vollständiger Umsatz der Ausgangsverbindung jedoch bereits nach 3 bis 7 Stunden erreicht.

Die Spaltung der Ester der Formel Ia zu dem Alkohol kann auf an sich bekannte Weise solvolytisch erfolgen. So können die Ester der Formel Ia unter zur Esterspaltung üblichen Bedingungen, z.B. durch basische Hydrolyse oder Alkoholyse gespalten werden. Beispielsweise können die Ester in Gegenwart einer anorganischen oder organischen Base mit Wasser oder einem niederen Alkohol oder deren Gemischen bei Temperaturen zwischen Raumtemperatur und Siedetemperatur des Reaktionsgemisches behandelt werden. Als anorganische Basen eignen sich z.B. Alkalimetallhydroxide oder -carbonate oder Erdalkalimetallhydroxide oder -carbonate, insbesondere Kalium- oder Natriumhydroxid. Die Spaltung kann direkt an dem nach der Hydrierung und Abtrennung von Katalysator und Lösungsmittel erhaltenem Rohprodukt ohne vorherige Reinigung oder Isolierung vorgenommen werden.

Sofern nötig, können die nach Abtrennung von Katalysator und Lösungsmittel als Hydrierungsprodukt oder nach einer daran anschließenden Esterspaltung gewonnenen rohen Verbindungen der Formel I auf an sich bekannte Weise, beispielsweise durch Destillation oder chromatographisch, gereinigt werden.

Das erfindungsgemäße Verfahren besitzt gegenüber der bisher bekannten Herstellung von Dihydronopol den Vorteil, daß bereits bei Temperaturen im Bereich der Raumtemperatur eine schnelle vollständige Umsetzung der Ausgangsverbindungen erfolgt und die cis-Verbindungen der Formel I mit guten Ausbeuten und hoher Stereoselektivität erhalten werden. So werden im allgemeinen Gesamtausbeuten an Verbindungen der Formel I von über 90 %, beispielsweise zwischen 95 und 100 %, erhalten. Das erfindungsgemäß hergestellte cis-Dihydronopol enthält im allgemeinen weniger als 0,4 %, häufig unter 0,2 % Verunreinigung durch trans-Dihydronopol.

Die Ausgangsester der Formel II können auf an sich bekannte Weise durch Acylierung des Alkohols der Formel III erhalten werden. Beispielsweise kann der Alkohol der Formel III unter zur Esterbildung üblichen Bedingungen mit reaktionsfähigen Säurederivaten der Formel IV

(s. Formel IV)

worin $R^1$ obige Bedeutung besitzt, und X Halogen oder einen $OCOR^1$-Rest, worin $R^1$ obige Bedeutung besitzt, bedeutet, umgesetzt werden. Das Acetat der Formel I kann auch auf an sich bekannte Weise direkt ausgehend von (-)-β-Pinen der Formel B erhalten werden, indem man dieses mit Formaldehyd in Eisessig umsetzt.

Die nachstehenden Beispiele sollen die Erfindung näher erläutern, ohne jedoch ihren Umfang einzuschränken.

Beispiel 1:

Herstellung von cis-Dihydronopol.

A) Zu einer Lösung von 100 g (-)-Nopol(= 0,6 Mol) und 130 ml Triäthylamin (= 0,93 Mol) in 600 ml Dichlormethan wurden unter Eiskühlung bei einer Temperatur von 5 bis 10 °C langsam 50 g Acetylchlorid (= 0,64 Mol) zugetropft. Anschließend wurde das Reaktionsgemisch noch 20 Minuten bei 5 bis 10 °C weitergerührt. Sodann wurde ohne Kühlung weitergerührt, bis das Reaktionsgemisch sich auf Raumtemperatur erwärmt hatte. Zur Aufarbeitung wurde das Reaktionsgemisch in 800 ml Eiswasser eingerührt. Die organische Phase wurde abgetrennt, die wäßrige Phase mit 100 ml Dichlormethan nachgewaschen und die vereinigten organischen Phasen mit gesättigter wäßriger Natriumhydrogencarbonatlösung neutral gewaschen und über Natriumsulfat getrocknet. Nach Abdestillieren des Dichlormethans verblieben 130 g Rohprodukt, aus welchem das (-)-Nopylacetat durch fraktionierte Destillation isoliert wurde. Es wurden 98 g (-)-Nopylacetat mit einem Siedepunkt von 117 bis 119 °C 13,3 mbar (10 mm Hg) und einem Brechungsindex $n^{20}_D$ = 1,4722 erhalten.

B) 20 g (-)-Nopylacetat wurden in 150 ml Essigsäureäthylester bei Raumtemperatur gelöst. Die Lösung wurde in einem 1-Liter-Autoklaven mit 1 g pulverförmigem Platinoxidkatalysator (= Adamskatalysator, Platingehalt 82 %) versetzt. Das Reaktionsgemisch wurde in dem Autoklaven mehrmals mit Stickstoff gespült. Anschließend wurde bei Raumtemperatur unter einem Wasserstoffdruck von 80 bar hydriert. Nach ca. 3 Stunden war die Wasserstoffaufnahme beendet. Der Wasserstoff wurde abgelassen und der Autoklav wurde mehrmals mit Stickstoff gespült. Sodann wurde die Reaktionslösung vom Katalysator abfiltriert und im Rotationsverdampfer eingeengt. Es wurden 20,3 g rohes cis-Dihydronopylacetat mit einem Siedepunkt von 126-128 °C 13.3 mbar (10 mm Hg) und einem Brechungsindex $n^{25}_D$ = 1,4685 erhalten.

Zur Feststellung des Reinheitsgrades und des Isomerenverhältnisses wurde das erhaltene rohe cis-Dihydronopylacetat durch Gaschromatographie (= GC) analysiert.

Für die gaschromatographische Untersuchung wurde ein Gaschromatograph vom Typ Sichromat mit FID (= Flammenionisationsdetector) der Fa. Siemens mit einer 30 m langen Quarzkapillarsäule mit einem Innendurchmesser von 0,32 mm (= Fused-Silica-Säule, Typ WCOT column 123-1334 der Fa. J u. W Scientific) verwendet, die als stationäre Phase ein immobilisiertes Siliconmaterial (= Durabond DB 624 der Fa. J u. W Scientific) enthält. Als Trägergas wurde Helium mit einem Vordruck von 0,6 bar und einer Durchflußgeschwindigkeit von 30 cm/sec. eingesetzt. Es wurde mit einer Injektionstemperatur von 200 °C und einem Temperaturprogramm der Säule von 180 °C bis 230 °C mit einer Aufheizrate von 4 °C/min und einer Detektionstemperatur von 250 °C gearbeitet. Zur Analyse wurden 0,5 Mikroliter einer 1 %-igen Lösung der Substanz in Methanol in den Gaschromatographen injiziert, welcher mit einem Split von 1 : 25 arbeitet. Die Analyse ergab folgende Werte:

| | |
|---|---|
| Gesamtgehalt an Dihydronopylacetat: | 98,1 % |
| cis/trans-Verhältnis: | 99,8 zu 0,2 |
| Gehalt an unhydriertem Ausgangsprodukt: | 0,3 % |

C) 20 g des unter B) erhaltenen rohen cis-Dihydronopylacetat wurden zu einer Lösung von 6,5 g Kaliumhydroxid in 30 ml Methanol getropft und das Reaktionsgemisch wurde 2 Stunden unter Stickstoffatmosphäre bei Raumtemperatur gerührt. Anschließend wurden dem Reaktionsgemisch 150 ml Äther zugesetzt und das erhaltene Gemisch wurde 3 mal mit je 75 ml Wasser gewaschen. Die vereinigten Waschwässer wurden mit 75 ml Äther extrahiert und die vereinigten Ätherphasen wurden über Natriumsulfat getrocknet. Nach Abdestillation des Äthers wurden 15,1 g cis-Dihydronopol als farbloses Öl mit einem Siedepunkt von 123 bis 125 °C 13.3 mbar (10 mm Hg) und einem Brechungsindex $n^{25}_{D}$ = 1.4882 erhalten.

Beispiel 2:

Herstellung von cis-Dihydronopylacetat.

2,5 kg (-)-Nopylacetat (hergestellt analog Beispiel 1A) wurden in 15 l Essigsäureäthylester gelöst und die Lösung in einen 30 l-Autoklaven gegeben. Anschließend wurde eine Aufschlämmung von 90 g pulverförmigem Platinoxidkatalysator (= Adamskatalysator) in 3 l Essigsäureäthylester zugesetzt. Der Autoklav wurde mehrmals mit Stickstoff gespült. Sodann wurde Wasserstoff mit einem Wasserstoffdruck von 95 bar in den Autoklaven gegeben, und es wurde bei diesem Wasserstoffdruck 135 min. bei einer Temperatur von 13 bis 23 °C hydriert. Sodann wurde der Wasserstoff abgelassen und der Autoklav mehrmals mit Stickstoff gespült. Das Reaktionsgemisch wurde aus dem Autoklaven abgelassen und von dem Katalysator abfiltriert. Der Autoklav wurde mit 3 l Äthylacetat nachgespült und der Katalysator ebenfalls mit dieser Spülflüssigkeit nachgewaschen. Die vereinigten Filtrate wurden eingeengt. Es wurden 2,456 kg rohes cis-Dihydronopylacetat (Siedepunkt 126 -128 °C 13.3 mbar (10 mm Hg), Brechungsindex $n^{25}_{D}$ = 1,4688) erhalten, welches gemäß der in Beispiel 1B beschriebenen gaschromatographischen Untersuchung die folgende Zusammensetzung besitzt:

| | |
|---|---|
| cis-Dihydronopylacetat | 98,7 % |
| trans-Dihydronopylacetat | 0,2 % |
| nicht umgesetztes (-)-Nopylacetat | 0,1 % |
| cis-Dihydronopol | 0,2 % |
| sonstige leicht flüchtige Bestandteile | 0,8 % |

Dies entspricht einer Gesamtausbeute an Dihydronopylacetat von 2,429 kg ≙ 96,2 mit einem cis/trans-Verhältnis von 99,8 zu 0,2.

Das Produkt wurde wie in Beispiel 1C beschrieben in das cis-Dihydronopol (Siedepunkt 123 - 125 °C 13.3 mbar (10 mm Hg) überführt.

Beispiel 3;

Herstellung von cis-Dihydronopylacetat

150 g (-)-Nopylacetat wurden in einem Autoklaven ohne Zusatz eines weiteren Lösungsmittels mit 5 g Adamskatalysator versetzt und wie in Beispiel 1 beschrieben hydriert. Die Wasserstoffaufnahme war nach ca. 6 Stunden beendet. Nach Spülen mit Stickstoff wurde das Hydrierungsprodukt zur Aufarbeitung mit 0,5 l Essigsäureäthylester verdünnt und von dem Katalysator abfiltriert. Dieser wurde nochmals mit 50 ml Essigsäureäthylester nachgewaschen. Filtrat und Waschflüssigkeit wurden vereinigt und das Lösungsmittel abdestilliert. Es wurden 151 g rohes cis-Dihydronopylacetat mit einem Brechungsindex $n^{25}_{D}$ = 1.4689 erhalten.

GC-Analyse:

GC-Analyse:
Gesamtgehalt an Dihydronopylacetat: 98,6 %
cis/trans-Verhältnis: quantitativ cis-Verbindung *)
Gehalt an unhydriertem Ausgangsprodukt: 0,2 %.

Beispiel 4:

Herstellung von cis-Dihydronopylacetat.

20 g (-)-Nopylacetat wurden in 150 ml Äthanol bei Raumtemperatur gelöst. Die Lösung wurde in einem 1-l-Autoklaven mit 1 g pulverförmigem Ruthenium/Kohle-Katalysator (Rutheniumgehalt 5 %) versetzt. Das Reaktionsgemisch wurde in dem Autoklaven mehrmals mit Stickstoff gespült. Anschließend wurde bei Raumtemperatur unter einem Wasserstoffdruck von 95 bar während 7 Stunden hydriert. Sodann wurde der Wasserstoff abgelassen und der Autoklav mehrmals mit

*) quantitativ = nach der vorstehend beschriebenen GC-Analysenmethode kein trans-Isomeres nachweisbar.

Stickstoff gespült. Die Reaktionslösung wurde vom Katalysator abfiltriert und im Rotationsverdampfer eingeengt. Es wurden 20,3 g rohes cis-Dihydronopylacetat mit einem Siedepunkt von 126 - 128 °C 13.3 mbar (10 mm Hg) und einem Brechungsindex $n^{25}_D$ = 1,4682 erhalten.

GC-Analyse:
Gesamtgehalt an Dihydronopylacetat: 98,9 %
cis/trans-Verhältnis: 99,6 : 0,4
Gehalt an unhydriertem Ausgangsprodukt: 0,2 %.

Beispiel 5:

Herstellung von cis-Dihydronopylacetat.

(-)-Nopylacetat wurde analog zu den in Beispielen 1 bis 4 beschriebenen Methoden unter den in der nachstehenden Tabelle I angegebenen Reaktionsbedingungen bei Raumtemperatur hydriert. Spätestens nach 3 Stunden wurde die Hydrierung abgebrochen, vom Katalysator abfiltriert, eingeengt und das erhaltene Hydrierungsprodukt gaschromatographisch nach der in Beispiel 1B angegebenen Methode analysiert. In der Tabelle I werden außer den Reaktionsbedingungen noch der nach der Reaktionszeit von 3 Stunden erzielte Umsatz an Ausgangsprodukt sowie das cis/trans-Verhältnis in dem Endprodukt angegeben.

Tabelle I

| Bsp. Nr. | Katalysator g/20g Edukt | Lösungsmittel | $H_2$-Druck in bar | Umsatz = % umgesetztes Ausgangsprod. | cis/trans-Verhältnis im Endprodukt |
|---|---|---|---|---|---|
| 5a** | 1g $PtO_2$ | Ethylacetat | 95-100 | 99,9 | 99,83:0,17 |
| 5b | 1g $PtO_2$ | Ethylacetat | 70 | 99,7 | 99,80:0,20 |
| 5c | 1g $PtO_2$ | Ethylacetat | 50 | 99,9 | 99,82:0,18 |
| 5d | 1g $PtO_2$ | Ethylacetat | 30 | 85,1 | 99,78:0,22 |
| 5e | 1g $PtO_2$ | n-Hexan | 80 | 86,2 | 99,88:0,12 |
| 5f | 1g $PtO_2$ | Toluol | 80 | 90,1 | 99,77:0,23 |
| 5g | 1g $PtO_2$ | Tetrahydrofuran | 80 | 97,1 | quantitativ* cis |
| 5h | 0,67g $PtO_2$ | -- | 80 | 89,7 | " " |
| 5i | 1g $PtO_2$ | Aceton | 80 | 92,2 | " " |
| 5j | 1g $PtO_2$ | $C_2H_5OH$ | 80 | 98,7 | " " |
| 5k | 1g $PtO_2$ | $CH_3OH$ | 80 | 97,4 | " " |
| 5l | 1g $PtO_2$ | $(CH_3)_2CHOH$ | 80 | 99,8 | " " |
| 5m | 1gPt/C(5%) | $C_2H_5OH$ | 80 | 86,4 | 99,66:0,34 |
| 5n *** | 1g $PtO_2$ | Ethylacetat | 95 | 99,7 | 99,85:0,15 |
| 5o**** | 1gRu/C(5%) | $C_2H_5OH$ | 95 | 95,3 | 99,59:0,41 |

* quantitativ cis = kein trans-Produkt nachweisbar, d.h. trans <<0,1 %

** Reaktionszeit nur 2,5 h

*** Reaktionszeit nur 1 h

**** Reaktionszeit 4 h, Reaktionstemperatur 50 °C

Beispiel 6:

Herstellung von cis-Dihydronopylpropionat.

A) 75 g (-)-Nopol wurden analog Beispiel 1A zusammen mit 97 ml Triäthylamin in 500 ml Dichlormethan gelöst und die Lösung wurde mit 40,9 g Propionsäurechlorid umgesetzt. Das Reaktionsgemisch wurde wie in Beispiel 1A beschrieben aufgearbeitet. Nach 2-facher fraktionierter Destillation wurden 54,6 g Nopylpropionat mit einem Siedepunkt von 136 bis 138 °C 13.3 mbar (10 mm Hg) erhalten.

B) 20 g Nopylpropionat wurden nach der in Beispiel 1B beschriebenen Methode bei Raumtemperatur und einem Wasserstoffdruck von 80 bar in Gegenwart von Adamskatalysator in Äthanol hydriert. Die Hydrierung wurde nach 3 Stunden abgebrochen und das Reaktionsgemisch wie in Beispiel 1B beschrieben aufgearbeitet. Es wurden 20,5 g rohes cis-Dihydronopylpropionat mit einem Brechungsindex $n^{20}_D$ = 1,4668 erhalten.

Das erhaltene rohe cis-Dihydronopylpropionat wurde gaschromatographisch analog der in Beispiel 1 B) beschriebenen Methode, jedoch unter Verwendung einer als stationäre Phase ein Polysiliconmaterial vom Typ CP-Sil 43CB (0,2 Mikron, organische Reste 50 % Methyl-, 25 % Cyanopropyl-, 25 % Phenylgruppen) der Fa. Chrompack enthaltenden 25 m langen Trennsäule mit einem Innendurchmesser von 0,32 mm (Säulentyp Fused Silica WCOT column 7745 der Fa. Chrompack), einer Injektortemperatur von 200 °C, einem Temperaturprogramm der Säule von 160 -220 °C mit einer Aufheizrate von 3 °C/min. analysiert. Die Analyse ergabe folgende Werte:

| | |
|---|---|
| Gesamtgehalt an Dihydronopylpropionat | 94,1 % |
| cis-/trans-Verhältnis | 99,9 zu 0,1 |
| Gehalt an unhydriertem Ausgangsprodukt | 1,3 % |

Das erhaltene rohe cis-Dihydronopylpropionat, kann wie in Beispiel 1C beschrieben in das cis-Dihydronopol überführt werden.

Beispiel 7:

Herstellung von cis-Dihydronopylbenzoat.

A) 49,9 g (-)-Nopol wurden analog Beispiel 1A zusammen mit 65 ml Triäthylamin in 350 ml Dichlormethan gelöst und das Reaktionsgemisch mit 43,3 g Benzoylchlorid umgesetzt. Anschließend wurde das Reaktionsgemisch analog Beispiel 1A aufgearbeitet. Nach fraktionierter Destillation wurden 51,1 g Nopylbenzoat mit einem Siedepunkt von 179 bis 182 °C 1.33 mbar (1 mm Hg) erhalten.

B) 26 g Nopylbenzoat wurden analog Beispiel 1B in 150 ml Äthanol gelöst, in Gegenwart von 1 g Adamskatalysator bei Raumtemperatur und 80 bar Wasserstoffdruck hydriert. Die Hydrierung wurde nach 8 Stunden abgebrochen und das Reaktionsgemisch wurde analog Beispiel 1B aufgearbeitet. Es wurden 25,4 g rohes cis-Dihydronopylbenzoat mit einem Brechungsindex $n^{20}_D$ = 1,5295 erhalten. Dieses wurde analog Beispiel 6B gaschromatographisch untersucht.

Gaschromatographische Analyse:

| | |
|---|---|
| Gesamtgehalt an Dihydronopylbenzoat | 92,4 % |
| cis/trans-Verhältnis | 99,8 zu 0,2 |
| Gehalt an unhydriertem Ausgangsmaterial | 3,3 |

Das erhaltene rohe cis-Dihydronopylbenzoat kann wie in Beispiel 1C beschrieben in das cis-Dihydronopol überführt werden.

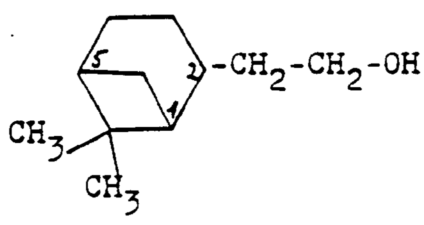

A

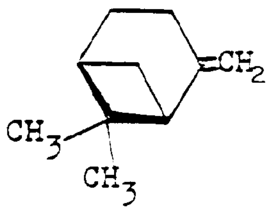

B

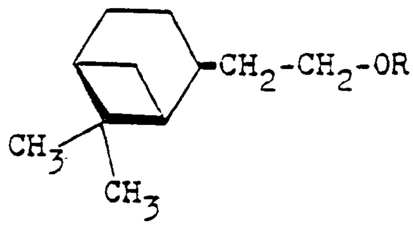

I

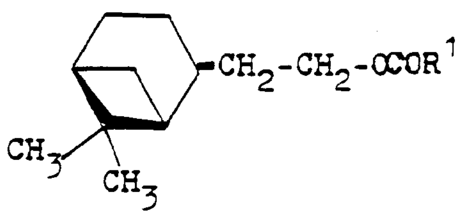

Ia

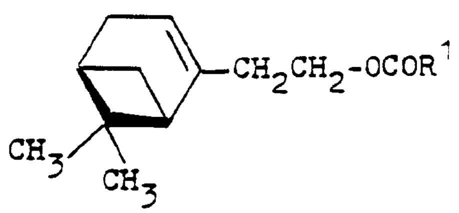

II

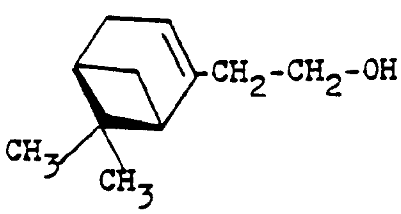

III

$R^1$-CO-X IV

**Patentansprüche**

1. Verfahren zur Herstellung von 2-((1S,2S,5S)-6,6-Dimethylbicyclo[3.1.1]hept-2-yl)-äthanolderivaten der allgemeinen Formel I

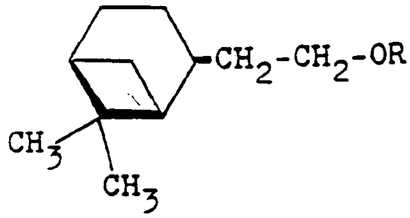

I

worin R Wasserstoff oder eine Acylgruppe $COR^1$, worin $R^1$ niederes Alkyl oder gegebenenfalls durch niederes Alkyl, niederes Alkoxy oder Hydroxy substituiertes Phenyl bedeutet, darstellt, dadurch gekennzeichnet, daß man Ester der allgemeinen Formel II

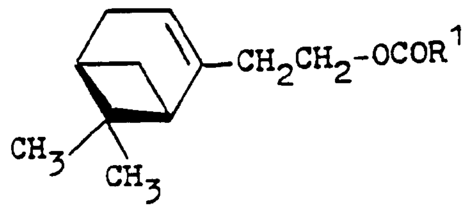

II

worin $R^1$ obige Bedeutung besitzt, mit Wasserstoff in Gegenwart eines festen Platin- oder Platinoxidkatalysators oder Ruthenium/Kohle-Katalysators hydriert zu Verbindungen der allgemeinen Formel Ia

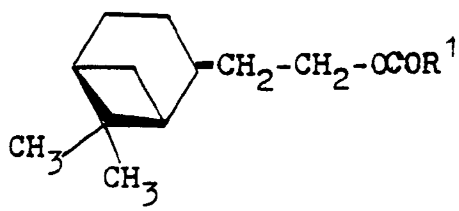

Ia

worin $R^1$ obige Bedeutung besitzt, und aus den Verbindungen der Formel Ia gewünschtenfalls die $COR^1$-Gruppe abspaltet.

2. Verfahren nach Anspruch 1, dadurch gekennzeichnet, daß die $COR^1$-Gruppe Acetyl ist.

3. Verfahren nach Anspruch 1, dadurch gekennzeichnet, daß die Hydrierung bei Temperaturen zwischen 15 und 50 °C, vorzugsweise 18 und 25 °C, durchgeführt wird.

## Claims

1. A process for the preparation of 2-((1S,2S,5S)-6,6-dimethylbicyclo[3.1.1]hept-2-yl)- ethanol derivatives of the general formula I

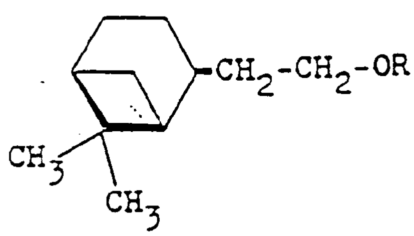

I

in which R represents hydrogen or an acyl group $COR^1$ in which $R^1$ denotes lower alkyl or phenyl which is option- ally substituted by lower alkyl, lower alkoxy or hydroxy, characterised in that esters of the general formula II

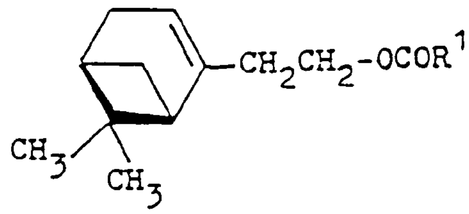

II

in which $R^1$ has the above meaning, are hydrogenated with hydrogen in the presence of a solid platinum or platinum oxide catalyst or ruthenium/carbon catalyst to give compounds of the general formula Ia

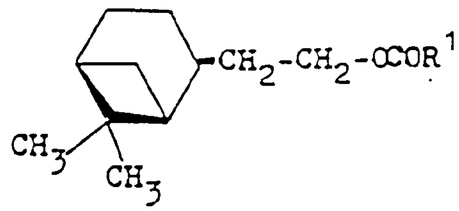

Ia

in which $R^1$ has the above meaning, and, if desired, the $COR^1$ group is cleaved off from the compounds of Formula Ia.

2. A process according to Claim 1, characterised in that the $COR^1$ group is acetyl.

3. A process according to Claim 1, characterised in that the hydrogenation is carried out at temperatures between 15 and 50°C, preferably 18 and 25°C.

## Revendications

1. Procédé pour la préparation de dérivés de 2-((1S,2S,5S)-6,6-diméthylbicyclo[3.1.1]hept-2-yl)-éthanol de formule générale I

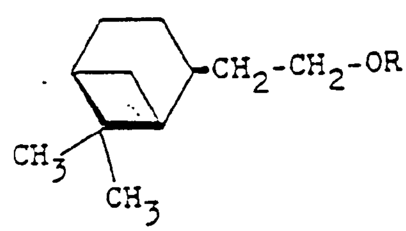

I

dans laquelle R représente un atome d'hydrogène ou un groupe acyle $COR^1$ dans lequel $R^1$ représente un groupe alkyle inférieur ou un groupe phényle éventuellement substitué par un radical alkyle inférieur, alcoxy inférieur ou hydroxy, caractérisé en ce que l'on soumet à une hydrogénation des esters de formule générale II

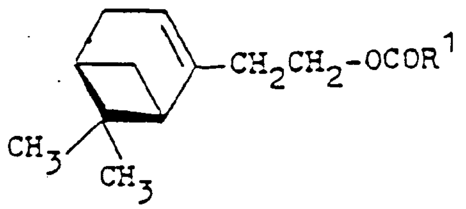

II

dans laquelle $R^1$ a la signification donnée ci-dessus, avec de l'hydrogène en présence d'un catalyseur solide à base de platine ou d'oxyde de platine, ou d'un catalyseur solide constitué de ruthénium/charbon, pour aboutir aux composés de formule générale Ia

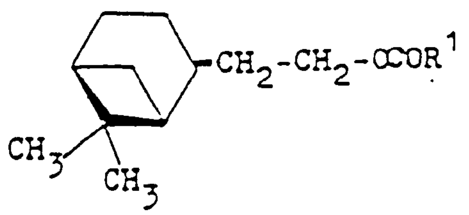

Ia

dans laquelle $R^1$ a la signification donnée plus haut, et, si on le désire, on élimine le groupe $COR^1$ des composés de formule Ia.

2. Procédé selon la revendication 1, caractérisé en ce que le groupe $COR^1$ est le groupe acétyle.

3. Procédé selon la revendication 1, caractérisé en ce que l'hydrogénation est effectuée à des températures comprises entre 15 et 50°C, de préférence entre 18 et 25°C.